# EUROPEAN PATENT SPECIFICATION

(11) **EP 3 911 291 B1**
(45) Date of publication and mention of the grant of the patent: **27.09.2023**
(21) Application number: 20702169.2
(22) Date of filing: 13.01.2020
(51) Int. Cl.: A61H 15/00, A61H 15/02

(54) **INTEGRATED DEVICE FOR THE NON-INVASIVE PERSONALISED TREATMENT OF SKIN BLEMISHES AND ASSOCIATED MULTI-STEP METHOD**
INTEGRIERTE VORRICHTUNG ZUR NICHTINVASIVEN, PERSONALISIERTEN BEHANDLUNG VON HAUTMAKELN UND ZUGEHÖRIGES MEHRSTUFIGES VERFAHREN
DISPOSITIF INTÉGRÉ SERVANT AU TRAITEMENT PERSONNALISÉ NON INVASIF D'IMPERFECTIONS CUTANÉES ET PROCÉDÉ EN PLUSIEURS ÉTAPES ASSOCIÉ

(30) Priority: 16.01.2019 IT 201900000679
(43) Date of publication of application: 24.11.2021
(73) Proprietor: Fenix Group S.r.l., 65015 Montesilvano (PE) (IT)
(72) Inventor: CAVALLETTI, Gianluca, 65015 Montesilvano (PE) (IT)
(74) Representative: Fiammenghi, Eva
(86) International application number: PCT/IB2020/050221
(87) International publication number: WO 2020/148624

(56) References cited:
- EP-A1- 2 361 598
- WO-A1-2007/122656
- CN-A- 106 038 208
- IT-A1- UB20 161 062
- US-A- 5 685 827
- US-A1- 2005 261 584

## Description

### Field of the art

The present invention regards the field of aesthetic and sports medicine as well as rehabilitation. In particular it describes a device suitable to recognize and treat, by means of compressive microvibration, some diseases such as cellulite, tissue recompacting, lymphatic and venous stasis, edematosis, muscle and cicatricial contractures, shoulder-neck syndrome and pubalgias. Other fields of application of this device are pre-post-surgery treatments for athletes, sports massage, lymphatic drainage and the treatment of pains of various origins.

### Prior art

The so-called cellulite, also called "gynoid lipodystrophy", "fibrous oedematous panniculopathy", "*adiposis edematosa*", "sclerotic fibrous oedematous panniculopathy", "*dermopanniculosis deformans*", "*status protrusions cutis*, and "liposclerosis", is a topographical manifestation of the skin associated with depressions or introflections frequent in pelvic and abdominal areas, hips, glutei and thighs. It may also manifest itself associated with nodules in subcutaneous adipose tissue and, in rare cases, with a suspected inflammatory condition.

The great confusion about the name to give to cellulite reflects the difficulty of correctly understanding the nature of the phenomenon. Despite the great interest in the treatment of this condition and the huge market for topical treatments aimed at improving its appearance, cellulite is still an enigmatic condition for medical researchers.

The high incidence (80-90%) in the female population presenting cellulite blemishes in post pubertal age, in addition to the fact that it occurs without symptoms, makes it to be considered a normal physiological condition. It can instead be considered in a pathological framework pointing out that whereas it is true that it is a normal condition for many women, it does not apply to all, and that in its most serious forms it manifests itself with nodules painful to palpation which lead to suspicion of inflammatory processes. Furthermore, it is generally associated with an excessive accumulation of subcutaneous adipose tissue whose relationship with cardiovascular risk is unclear.

Dozens of different causes have been hypothesised, including: metabolism disorders, nutritional disorders, specific subcutaneous architectures, alterations of connective tissue structure, hormonal factors, genetic factors, microcirculatory system disorders, alterations in the lymphatic system, alterations in the extracellular matrix, inflammations.

Cellulite classifications are as follows:
- oedematous: i.e. associated with an accumulation of liquids, especially around ankles, calves, thighs and arms.
- fibrous: i.e. associated with an increase in trabecular structures and connective tissue septa which divide the subcutaneous adipose tissue into various lobes; fibrous cellulite is characterised by small nodules that are not perceivable to the touch except as a subcutaneous roughness and by the orange peel-like skin.
- sclerotic: i.e. associated with a hardening of the tissues associated with large nodules and plaques; this form can be very painful.

Various measures can help prevent cellulite. Some attention in nutrition, constant physical activity, the use of comfortable and not too tight clothes, shoes with not too high heels and changes in posture are the easiest remedies to implement to prevent this annoying disorder. The huge market of treatments for reducing cellulite ranges from topical products to liposuction. However, many of these treatments lack evidence of effectiveness. Below is a list of the most common ones:
- topical products i.e. creams and the like to be applied manually on the area in question constantly and on daily basis for long periods of time;
- massages;
- pressure therapy: treatment based on the external pressure of the patient's limbs or the abdominal area thereof through a specific apparatus;

- mesotherapy: a technique for the administration of drugs through the intraepidermal, superficial and deep intradermal and subcutaneous or hypodermic routes, has the advantages of being suitable for reducing doses of active ingredient which have a prolonged effect over time;
- emulsion-lipolysis: a pharmacological therapy which can be carried out by means of a regional injection with hypothalamic phospholipids, carnitine, aminophylline, lidocaine 2%, saline solution;
- lipoapoptosis: a pharmacological therapy that can be carried out by means of a regional injection with vitamin C, trivalent iron, lidocaine 2%, water for injectable preparations;
- carboxy therapy: treatment that originates from thermal medicine and consists in the administration of carbon dioxide through the subcutaneous route;
- osmotic lipoclasia: cutaneous biostimulation with platelet-rich plasma;
- oxygenoclasia: technique for introducing pure medical oxygen into the excess fat through a small needle;
- endermologie system: a technique designed in France in the 70s in which a vacuum system and a plurality of rollers, mounted on a handpiece, exert pressure and lifting actions on the skin causing a redistribution of subcutaneous fat.
- ultrasound therapy: treatment based on the effect produced by mechanical vibrations applied to the outer tissues of the body by means of an ultrasonic transducer.
- laser therapy: treatment that acts at various levels of depth based on the power of the transmitted rays.
- diathermy: a practice, without scientific validation, which supports curative properties induced by the increase in the temperature of some non-superficial areas of the body caused by the passage of electric current.

As observable from the list above, which is not exhaustive, the market is saturated with treatments, among which there are some without sure results. Ineffectiveness is often due to the fact that, as explained above, the cause of cellulite and of the disorders it entails remain unclear.

There are also various patents claiming treatments for cellulite care such as US 2002147467 which provides for, first of all, an exfoliating treatment to maximize the absorption of the substances applied to the skin, then the administration of mechanical vibrations associated with electric pulsations in the area in question.

EP 1844750, on the other hand, claims an instrument for aesthetic treatments, therefore not of medical nature, which reduces cellulite through ultrasonic vibrations with a frequency between 25 kHz and 50 kHz.

GB 2303552 discloses a medical apparatus capable of reducing cellulite through ultrasounds at a frequency of about 3.3 MHz reaching a depth of 1.27 - 2.54 cm from the skin surface without overheating the part.

The worldwide patent, WO2007122656, owned by Fenix S.r.l., a market leader in anti-cellulite and anti-blemishes therapies as well as skin disorders in general, claims a computer-controlled vibratory device, used to treat parts of the body, the whose primary characteristic is to apply massages by inducing mechanical oscillations by means of a plurality of massage elements which are rotatably mounted about their own axis in a roller driven by an electric motor. The rotation of the roller around the shaft thereof is controlled by the operator through an electronic control unit, by selecting a program suitable for the treatment to be carried out. The device described in this patent performs a massage based on the resistance opposed by the muscles located under the adipose layer.

Document ITUB20161062A1 discloses a compressive micro-vibration device comprising a first cylinder provided with a plurality of rotary balls arranged as a honeycomb, a second cylinder provided with a plurality of abrasive elements constituted by balls or ovals or rotary cylinders, an energy absorption sensor adapted to detect the resistance of the tissue and a software adapted to recognize the disturbance of the skin substrate and the anti-cellulite process.

An object of the present invention is to provide a device and a compressive microvibration method customised on the disorders suffered by each patient, which performs four actions synergistically by acting on the vascular system at the microcirculation level, on the lymphatic system, on the tissue re-compaction and on the analgesic effect, thus also entailing a muscular effect. The operation of this device relies on a sensor capable of detecting the type of disorder and a dedicated software that determines the best therapy to be followed case by case.

### Description of the invention

The present invention is directed to a integrated device for the non-invasive personalised treatment of skin blemishes according to claim 1.

Subsidiary aspects of the invention are provided in the dependent claims.

Advantageously, the device integrates handpieces to perform different treatments to be modulated depending on the disorder suffered by each patient. The treatment is particularly effective on muscle contractures, cellulite, stretch marks, wrinkles and more. The device comprises a portable console provided, at the upper part, with a plurality of recesses each suitable to house a corresponding handpiece.

The device comprises at least 3 handpieces and possibly a fourth additional handpiece. Said handpieces are the following:
- A first handpiece suitable for performing a percussion treatment on the body and/or face of a patient by means of a rotor comprising a plurality of rotating elements installed on a corresponding plurality of semi-axes installed equally spaced on the circumference of a ring nut. The rotor is included in a head of the handpiece, which has an opening protected by an elastic membrane designed to allow the percussion to be carried out, avoiding direct contact between the rotating elements and the patient's epidermis. Included in the handle of the handpiece, consisting of two half-handles suitable to be mutually engaged in this case, is the electric motor which controls the percussion frequency of said rotor. Said first handpiece is therefore advantageously suitable cause a microtrauma at the wrinkle furrow, thus facilitating the natural recovery and therefore a reduction of the wrinkle depth or of the stretch on which the percussion is applied.
   Furthermore, in the preferred embodiment said first handpiece has a movable head, tilted according to an angle of 10°, provided with a spot laser and a micro-camera.
- A second handpiece suitable for carrying out an ablative micro-vibration treatment on the patient's body, also obtaining an exfoliating effect. This object is achieved by means of a rotor comprising a plurality of rotating and vibrating elements suitable to vibrate and rotate in a converging or diverging direction with respect to a median axis, according to the arrangement of a plurality of pins on which said rotating elements are fitted. The median axis of the rotor is occupied by a middle ring nut on which the operator can advantageously act to cause said rotating elements to impart a converging or diverging force on the patient's skin. The rotor is inserted into a shell provided with an open side, which allows the contact between said rotating elements and the body of said patient, and with a closed side facing the operator, comprising a pair of light indicators suitable to signal the converging or diverging direction of the rotating elements to the operator. The rotor is connected beneath an electric motor, integrated in the handle of the handpiece, which determines the frequency of rotation and vibration of the rotor. Said second handpiece is therefore advantageously suitable to expand or compact the skin tissue by alleviating blemishes such as wrinkles, stretch marks and scars, thus also obtaining an exfoliating effect, referred to as "*stretching*" in the technical jargon. In the preferred embodiment of the present invention, this exfoliating effect is obtained by a surface treatment of the outer surface of each rotating element which has a predetermined roughness and density, or softness.
- A third handpiece suitable for performing a compressive micro-vibration treatment on the face of a patient by means of a rotor provided with rotating elements suitable to vibrate and rotate around a plurality of pins which extend from an upper ring nut to a lower ring nut. The rotor is inserted into a shell provided with an open side, which allows the contact between said rotating elements and the body of the patient, and with a closed side facing the operator, provided with a light pressure indicator which signals, to the operator, the pressure with which he/she is pressing on the patient's face. Also in the case of the third handpiece, the rotor is driven by an electric motor integrated in the handle. Thus, said third handpiece is advantageously suitable to expand the pores of the patient's epidermis.
- A fourth handpiece, present only optionally with respect to the device of the invention, suitable for carrying out a treatment for the vibro-transportation of active ingredients in depth on the body and/or face of a patient by means of a vibrating head suitable to vibrate, at a predetermined frequency driven by an electric motor integrated in the handle of the handpiece. Optionally, said fourth handpiece also comprises an active ingredient reservoir suitable to release the desired amount of cream, gel, or mousse on the patient's body upon pressing a special key by the operator. Using the fourth handpiece following the application of at least one of the first three handpieces allows an in-depth penetration of the desired active ingredient, which would otherwise be achievable only by injection, making the treatment non-invasive, painless, and at least equally effective.

All said handpieces are power-supplied by special electrical cables which connect them to said console. They can also be easily disconnected by operating special connection means which connect the distal ends of said power supply cables to the electric motors included in said handpieces.

Each handpiece also advantageously includes a rotation and vibration adjustment device and an electric energy absorption sensor. The latter is suitable to detect the amount of electric energy absorbed by each handpiece in order to obtain the desired rotation and vibration of the corresponding rotors, communicating the detected data to a control unit programmed with a dedicated software. By processing the data coming from said electric energy absorption sensor, the control unit acts, in real time, on the frequency adjustment devices of the handpiece in order to obtain an immediate variation of the rotation and vibration frequency of said rotors, within a pre-established range comprised between 20 Hz and 700 Hz, preferably between 40 Hz and 355 Hz, depending on the mechanical strength provided by the skin tissue of the patient at contact with the handpiece in use.

This device constitutes the main advantage offered by the present invention, given that the operator is greatly facilitated in the work thereof by the possibility of automatically and immediately identifying and modifying the intensity of the treatment in areas affected by different muscle contractures or tissue disorders.

All the data relating to the therapy in progress are displayed and can be modified manually by the operator through a communication interface, preferably integrated in said console. More in detail, a method of use of the device not forming part of the present invention consists of the following steps:
- setting the parameters, through said communication interface, based on the characteristics of the patient's tissue, the age and musculature thereof;
- a test, in which, by gripping a handpiece and placing the relative rotor at contact with the patient's body or face, the relative electric energy absorption sensor sends the detected data to the control unit integrated in the treatment device. By comparing the electric energy absorption data and the data of the vibrations and rotations frequency set by the operator, through the communication interface, the control unit communicates to the operator the correctness or incorrectness of the frequency range of the vibrations and rotations set;
- administering the therapy, in which the operator covers the areas of the patient's body or face to be treated with a handpiece at a time according to a sequence pre-established by the type of treatment. In the preferred embodiment, the recesses in which the handpieces are housed are advantageously provided with means for retaining said handpieces suitable for releasing the handpieces only in the order pre-established by the therapy being carried out. During the treatment, the operator applies a predetermined pressure perpendicular to the skin tissue of the patient and, should the electric energy absorption sensor, possibly combined with a pressure or a motor revolutions sensor, of the handpiece in use, detect an area of greater mechanical strength, for example a muscle contracture, said control unit, which processes the data sent by said electric energy absorption sensor, sends a command to increase the vibrations and rotations frequency to the frequency adjustment device of the handpiece in use, until a lower mechanical resistance is detected.
- At the end of therapy, in a fourth step of monitoring the homogeneity of the treatment on the area affected by the skin disorder or blemish, the operator examines the reddening of the skin due to the vascularization of the tissue induced by the treatment, so as to ensure that each area of the body or face has been treated uniformly. A more scientific version of this final step of the method of use of the machine provides for the presence of a temperature sensor for each handpiece, suitable to communicate, to the control unit of said device, the temperature variation of the skin tissues of the patient from the beginning of the treatment.

Other embodiments of the device which make it even more advantageous both in terms of efficiency and comfort for the operator and the patient, provide for the presence of at least one of the following devices:
- a resistor inside said handpieces, suitable to heat the portion of the handpiece which is at contact with the patient's body or face, up to a predetermined temperature;
- a resistor inside the recesses suitable to heat the corresponding handpieces before they are placed at contact with the patient's body or face;
- a device for disinfecting the handpieces by means of a common infrared lamp.

Lastly, the rotating elements of the handpieces can consist of balls, ovoids or cylinders, preferably balls, arranged honeycomb-like or V-like manner, made of anti-allergy silicone or gel of hardness comprised between 5 shore and 100 shore, preferably 35 shore.

In a variant embodiment of said rotating elements (balls) the effects of the treatment described so far, already very advantageous, are added to the further beneficial effects of the "micro-vacuum" by virtue of the fact that the outer surface of said rotating elements is provided with a plurality of spherical cap-shaped recesses suitable to create a suction effect when pressed onto the patient's epidermis.

The advantages provided by the present invention will be clear in light of the description outlined up to now. Furthermore, they will be more apparent due to the attached figures and relative detailed description.

### Description of the figures

The invention will be described hereinafter in at least one preferred embodiment, provided by way of non-limiting example, with reference to the attached figures, wherein:
- FIGURE 1 shows a three-dimensional view of the device in a three-recess embodiment 1.1-1.2-1.3. Also shown is the fourth handpiece 7, optionally present.

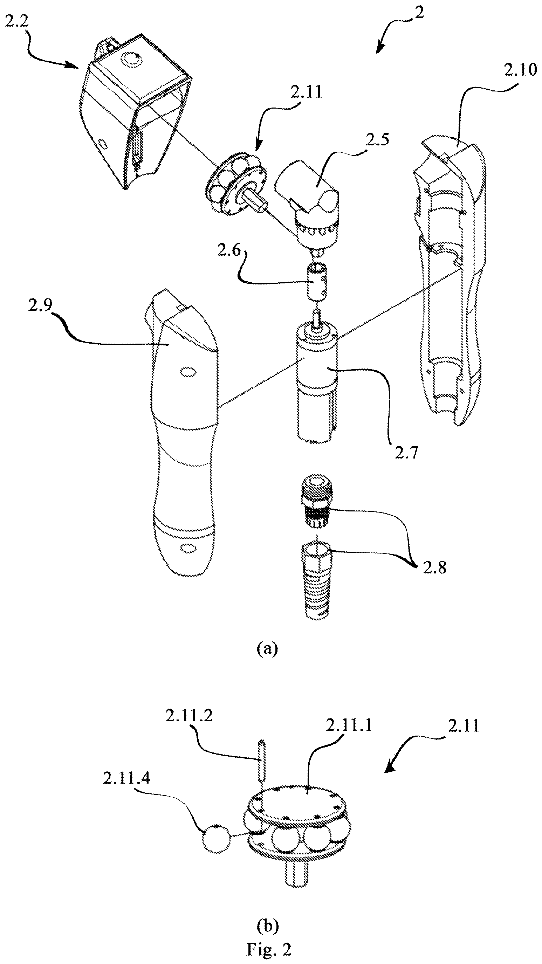

- FIGURE 2(a) illustrates an exploded view of the first handpiece 2.
- FIGURE 2(b) shows, in detail, an exploded view of the rotor 2.11 of the first handpiece 2.

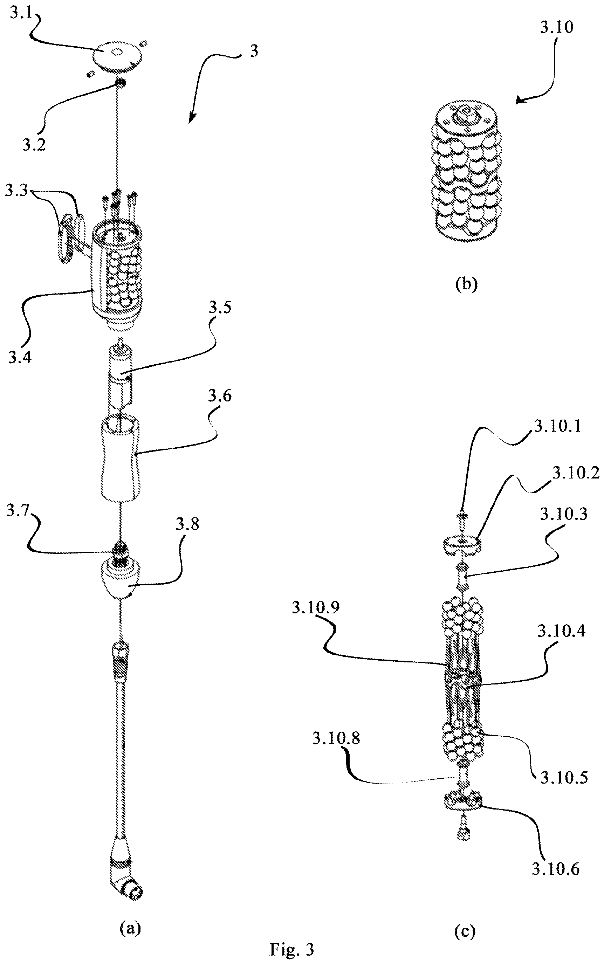

- FIGURE 3(a) shows an exploded view of the second handpiece 2.
- FIGURE 3(b) shows a three-dimensional view of the rotor 3.10 of the second handpiece 3.
- FIGURE 3(c) shows an exploded view of the aforementioned rotor 3.10.

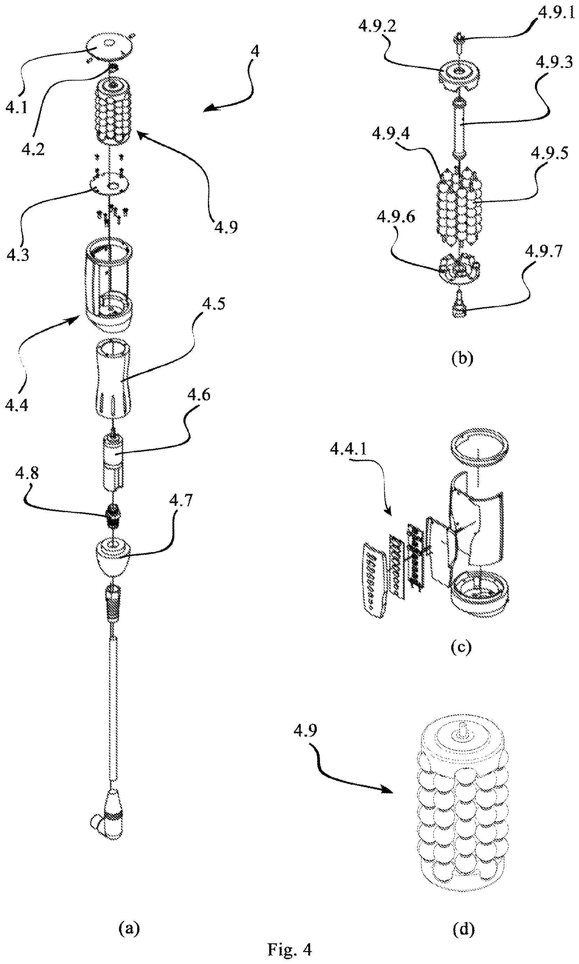

- FIGURE 4(a) illustrates an exploded view of the third handpiece 4.
- FIGURE 4(b) shows an exploded view of the rotor 4.9 of the third handpiece 4.
- FIGURE 4(c) shows an exploded view of the shell 4.4 of the third handpiece 4.
- FIGURE 4(d) shows a three-dimensional view of the aforementioned rotor 4.9.

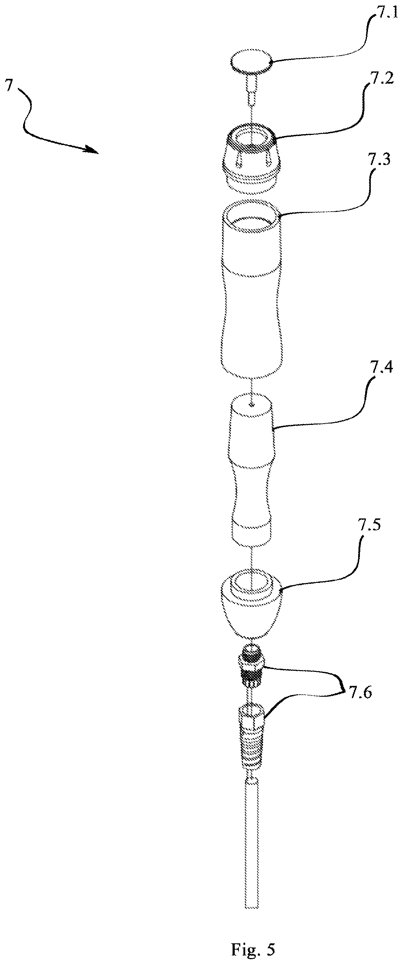

- FIGURE 5 shows an exploded view of the fourth handpiece 7.

### Detailed description of the invention

Now, the present invention will be illustrated purely by way of non-limiting or non-binding example, with reference to the figures illustrating some embodiments regarding the present inventive concept.

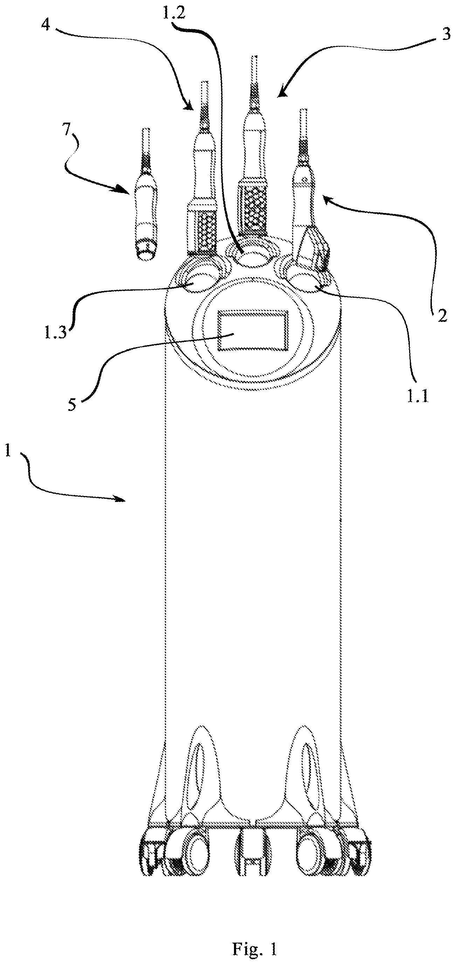

With reference to FIG. 1, shown in its entirety is the device subject of the present invention in which there is a portable console 1 containing a control unit (not visible) provided with a communication interface 5 and with three recesses 1.1-1.2-1.3, preferably heated, each of which is suitable to house a corresponding handpiece 2-3-4.

Depending on the patient's disorder, the operator selects a vibrations and rotations frequency range that the rotors 2.11-3.10-4.9 of the handpieces 2-3-4 will perform when placed on the patient's skin.

The correctness of such range is verified immediately by virtue of the fact that each handpiece 2-3-4 is provided with electric energy absorption sensor which communicates the detected data suitable to detect the amount of electric energy suitable to communicate the detected data to said control unit programmed with a dedicated software suitable to process the data coming from said electric energy absorption sensors, possibly combined with pressure or motor revolutions sensor, and to consequently act, in real time, on the frequency adjustment devices of each handpiece 2-3-4 in order to obtain an immediate variation of the rotation and vibration frequency of said rotors 2.11-3.10-4.9, within a pre-established range comprised between 20 Hz and 700 Hz, preferably between 40 Hz and 355 Hz, depending on the mechanical strength provided by the skin tissue of the patient at contact with one of said handpieces 2-3-4;

Subsequently, the operator can proceed with the therapy by covering the areas of the patient's body or face to be treated with a handpiece 2-3-4 at a time, for a pre-established time interval, applying a predetermined pressure perpendicular to the patient's skin tissue. In the recesses 1.1-1.2-1.3 of the console 1, there are retention means which release the handpieces 2-3-4 only in the order pre-established by the therapy being carried out, preventing any possibility of error.

Each handpiece 2-3-4 has a rotor 2.11-3.10-4.9 consisting of rotating elements 2.11.4-3.10.5-4.9.5 or balls arranged in a honeycomb-like or V-like manner, made of anti-allergy silicone or gel with a hardness equal to 35 shore.

In greater detail, the first handpiece 2 is suitable to perform a percussive treatment on the patient's body and/or face. The balls 2.11.4 of the rotor 2.11 are installed on a corresponding plurality of semi-axes 2.11.2 equally spaced on the circumference of a ring nut 2.11.1. The rotor 2.11 is installed between a movable head which allows the movement of the membrane 2.2 and an angular support 2.5. Said head 2.2 is provided with an elastic membrane suitable to allow the execution of the percussion preventing the direct contact between the balls 2.11.4 and the epidermis of the patient. The angular support 2.5, on the other hand, is fitted, by means of a suitable insert 2.6 on an electric motor 2.7 which controls the percussion frequency of said rotor 2.11, power-supplied by a special cable, connected through common connection means 2.8, connected to said console 1. The angular support 2.5, the insert 2.6, the electric motor 2.7 and the connection means 2.8 are arranged in a pair of half-handles 2.9-2.10, which are suitable to engage each other so as to constitute the grip handle of said first handpiece 2.

The second handpiece 3, is suitable for carrying out an ablative micro-vibration treatment on the patient's body. The balls 3.10.5 of the rotor 3.10 vibrate and rotate in a convergent or divergent direction according to the arrangement of a plurality of pins 3.10.9 into which the balls 3.10.5 are fitted. More in detail, said pins 3.10.9 extend from an upper ring nut 3.10.2 to a middle ring nut 3.10.4 and from said middle ring nut 3.10.4 to a lower ring nut 3.10.6. The rotation of said middle ring nut 3.10.4 by said operator modifies the configuration of said pins 3.10.9 in order to cause the balls 3.10.5 to converge or diverge toward or from said middle ring nut 3.10.4. The rotor 3.10 rotates around an upper semi-axis 3.10.3 and a lower semi-axis 3.10.8 and it is inserted into a shell 3.4 provided with an open side and a closed side. The open side allows contact between the balls 3.10.5 and the patient's body while the closed side is directed toward the operator and comprises a pair of light indicators 3.3 suitable to signal to the operator the converging or diverging direction of the balls 3.10.5 on the patient's body. The rotor 3.10 is connected, at the upper part by means of a pin 3.10.1, to a bearing 3.2 and lastly to an upper plug 3.1 of the second handpiece 3. At the lower part, on the other hand, the rotor 3.10 is connected to an electric motor 3.5, integrated in the handle 3.6, power-supplied by a special cable, connected by common connection means 3.7 included in a lower cap 3.8.

The third handpiece 4, is suitable for carrying out a compressive micro-vibration treatment on the patient's face. The balls 4.9.5 of the rotor 4.9 vibrate and rotate around a plurality of pins 4.9.4 which extend from an upper ring nut 4.9.2 to a lower ring nut 4.9.6. The rotor 4.9 rotates, at a pre-established frequency, around a rotation axis 4.9.3 and it is inserted into a shell 4.4 provided with an open side and a closed side. The open side allows the contact between said balls 4.9.5 and the body of said patient; the closed side is directed toward the operator and it comprises a light pressure indicator 4.4.1 suitable to signal, to the operator, the pressure with which he/she is pressing the handpiece 4 on the patient's face. The rotor 4.9 is connected, at the upper part by means of a pin 4.9.1, to a bearing 4.2 and lastly to an upper cap 4.1, whereas at the lower part it is connected, by means of a joint 4.9.7, to an electric motor 4.6, integrated in a handle 4.5, power-supplied by a special cable connected through common connection means 4.8 included in a lower cap 4.7.

At the end of the treatment, the operator verifies whether each tissue was treated homogeneously by monitoring the increase of skin temperature, due to the vascularization of the tissue induced by the treatment, performed by the control unit which processes the temperature data detected by a temperature sensor of the handpiece 2-3-4 in use.

Lastly, it is clear that the invention described up to now may be subjected to modifications, additions or variants obvious to a man skilled in the art, without departing from the scope of protection outlined by the attached claims.

## Claims

1. Integrated device for the non-invasive personalised treatment of skin blemishes, suitable to be operated by an operator in order to perform a treatment on the body and/or on the face of a patient for the reduction of muscle contractures, cellulite, stretch marks, wrinkles and others blemishes, said device comprising a portable console (1), provided, at the upper part, with a plurality of recesses (1.1-1.2-1.3) each suitable to house a corresponding handpiece (2-3-4) suitable to be grasped by said operator in order to perform a step of a pre-established treatment on the body and/or on the face of said patient; said handpieces (2-3-4) comprising:
- a first handpiece (2) suitable to perform a percussion treatment on the body and/or on the face of a patient by means of a rotor (2.11) comprising a plurality of rotating elements (2.11.4) installed on a corresponding plurality of equidistant semi-axes (2.11.2) on the circumference of a ring nut (2.11.1); said rotor (2.11) being installed between a movable head (2.2), provided with spot laser and with micro-camera, of said first handpiece (2) and an angular support (2.5); said head (2.2) being provided with an elastic membrane suitable to allow to perform the percussion, avoiding the direct contact between the rotating elements (2.11.4) and the epidermis of the patient; said angular support (2.5) being fitted, by means of a suitable insert (2.6), on an electric motor (2.7) which controls the percussion frequency of said rotor (2.11); said electric motor (2.7) being power supplied by a special cable, connected to said first handpiece (2) by means of common connection means (2.8), connected to said console (1); said angular support (2.5), said insert (2.6), said electric motor (2.7) and said connection means (2.8) being arranged in a pair of semi-handles (2.9-2.10) suitable to be mutually engaged in order to form the handle for grasping said first handpiece (2); said first handpiece (2) being suitable to cause a micro-trauma at the wrinkle furrow, thus facilitating the natural recovery and hence a reduction of the wrinkle depth;
- a second handpiece (3) suitable to perform an ablative micro-vibration treatment on the body of the patient by means of a rotor (3.10) comprising a plurality of rotating elements (3.10.5) suitable to vibrate and rotate in a mutually converging or diverging direction depending on the arrangement of a plurality of pins (3.10.9) on which said rotating elements (3.10.5) are fitted; said pins (3.10.9) extending from an upper ring nut (3.10.2) to a middle ring nut (3.10.4) and from said middle ring nut (3.10.4) to a lower ring nut (3.10.6); the rotation of said middle ring nut (3.10.4) by said operator being suitable to modify the configuration of said pins (3.10.9) in order to ensure that said rotating elements (3.10.5) converge or diverge toward or from said middle ring nut (3.10.4); said rotor (3.10) being suitable to rotate, at a pre-established frequency, around an upper semi-axis (3.10.3) and a lower semi-axis (3.10.8); said rotor (3.10) being inserted into a shell (3.4) provided with an open side and with a closed side; said open side allowing the contact between said rotating elements (3.10.5) and the body of said patient; said closed side being directed toward the operator and comprising a pair of light indicators (3.3) suitable to signal to the operator the converging or diverging direction of the rotating elements (3.10.5) on the body of the patient; said rotor (3.10) being connected, at the top part by means of a pin (3.10.1), to a bearing (3.2) and finally to an upper cap (3.1) of said second handpiece (3); said rotor (3.10) being connected, at the lower part, to an electric motor (3.5) integrated in a handle (3.6); said electric motor (3.5) being suitable to determine the rotation and vibration frequency of said rotor (3.10) and being power supplied by a special cable, connected to said second handpiece (3) by means of common connection means (3.7) included within a lower cap (3.8) connected to said handle (3.6), said power supply cable being connected to said console (1); said second handpiece (3) being suitable to expand or compact the skin tissue, alleviating blemishes such as wrinkles, stretch marks and scars, also obtaining an exfoliating effect called "stretching";
- a third handpiece (4) suitable to perform a compressive micro-vibration treatment on the face of a patient by means of a rotor (4.9) comprising a plurality of rotating elements (4.9.5) suitable to vibrate and rotate around a plurality of pins (4.9.4) on which said rotating elements (4.9.5) are fitted; said pins (4.9.4) extending from an upper ring nut (4.9.2) to a lower ring nut (4.9.6); said rotor (4.9) being suitable to rotate, at a pre-established frequency, around a rotation axis (4.9.3); said rotor (4.9) being inserted into a shell (4.4) provided with an open side and with a closed side; said open side allowing the contact between said rotating elements (4.9.5) and the body of said patient; said closed side being directed toward the operator and comprising a light pressure indicator (4.4.1) suitable to signal to the operator the pressure with which he/she is pressing the handpiece (4) on the face of the patient; said rotor (4.9) being connected, at the upper part by means of a pin (4.9.1), to a bearing (4.2) and finally to an upper cap (4.1) of said third handpiece (4); said rotor (4.9) being connected, at the lower part by means of a joint (4.9.7), to an electric motor (4.6) integrated in a handle (4.5); said electric motor (3.5) being suitable to determine the rotation and vibration frequency of said rotor (4.9) and being power supplied by a special cable, connected to said third handpiece (4) by means of common connection means (4.8) included within a lower cap (4.7) connected to said handle (4.5), said power supply cable being connected to said console (1); said third handpiece (4) being suitable to expand the pores of the epidermis of the patient;
- at least one rotation and vibration adjustment device, integrated in each handpiece (2-3-4), suitable to control the frequency of said rotations and vibrations of said rotors (2.11-3.10-4.9) of said handpieces (2-3-4);
- at least one electric energy absorption sensor for each handpiece (2-3-4), suitable to detect the amount of electric energy absorbed by each handpiece (2-3-4) in order to obtain the desired rotation and vibration of the relative rotors (2.11-3.10-4.9); said electric energy absorption sensor being suitable to communicate the detected data to
- a control unit programmed with a dedicated software suitable to process the data coming from said electric energy absorption sensors and to consequently act, in real time, on said frequency adjustment devices of each handpiece (2-3-4) in order to obtain an immediate variation of the rotation and vibration frequency of said rotors (2.11-3.10-4.9), within a pre-established range comprised between 20 Hz and 700 Hz, preferably between 40 Hz and 355 Hz, depending on the mechanical strength provided by the skin tissue of the patient at contact with one of said handpieces (2-3-4);
- a communication interface (5), preferably integrated in said console (1), suitable to allow an operator to receive in real time an information regarding the treatment underway and to set the respective parameters, including the frequency of the vibrations and of the rotations to be administered to the patient through said handpieces (2-3-4),
wherein the device further comprises means for retaining said handpieces (2-3-4-7) suitable to release the constraint between a recess (1.1-1.2-1.3) and the corresponding handpiece (2-3-4-7) only in the order pre-established by the therapy that is being performed.

2. Integrated device for the non-invasive personalised treatment of skin blemishes, according to the preceding claim 1, **characterised in that** it comprises at least one fourth handpiece (7) suitable to perform a treatment for the vibro-transportation of active ingredients deep into the body and/or on the face of a patient by means of a head (7.1) suitable to vibrate by exploiting square-wave electrical pulses, at a pre-established frequency driven by an electric motor (7.4) inserted into a handle (7.3) connected, at the upper part, to a support (7.2) from which said vibrating head (7.1) projects above; said electric motor (7.4) being power supplied by a special cable, connected to said fourth handpiece (7) by means of common connection means (7.6) included within a lower cap (7.5) connected to said handle (7.3), said power supply cable being connected to said console (1).

3. Integrated device for the non-invasive personalised treatment of skin blemishes, according to the preceding claim 2, **characterised in that** said fourth handpiece (7) comprises an active ingredient reservoir suitable to release the desired amount of cream, gel, or mousse on the body of the patient upon pressing a suitable key by said operator.

4. Integrated device for the non-invasive personalised treatment of skin blemishes, according to any one of the preceding claims, **characterised in that** said handpieces (2-3-4-7) are provided with an internal resistor suitable to heat the portion of said handpiece (2-3-4-7) that is at contact with the body or with the face of the patient when said handpiece (2-3-4-7) is in use, up to a pre-established temperature.

5. Integrated device for the non-invasive personalised treatment of skin blemishes, according to any one of the preceding claims 1 to 3, **characterised in that** each recess (1.1-1.2-1.3) is provided with an internal resistor suitable to heat the corresponding handpieces (2-3-4-7) before they are placed at contact with the body or with the face of the patient.

6. Integrated device for the non-invasive personalised treatment of skin blemishes, according to any one of the preceding claims, **characterised in that** each handpiece (2-3-4-7) is provided with a temperature sensor suitable to communicate, to the control unit of said device, the temperature variation of the skin tissues of the patient from the start of the treatment, said control unit communicating the value of said variation by means of said communication interface (5).

7. Integrated device for the non-invasive personalised treatment of skin blemishes, according to any one of the preceding claims, **characterised in that** said rotating elements (2.11.4-3.10.5-4.9.5) of said handpieces (2-3-4) consist of balls, ovoids or cylinders, arranged to form a honeycomb-like or V-like shape; said rotating elements (2.11.4-3.10.5-4.9.5) being made of non-allergenic silicone or of gel with hardness comprised between 5 shore and 100 shore, preferably 35 shore.

8. Integrated device for the non-invasive personalised treatment of skin blemishes, according to any one of the preceding claims, **characterised in that** said rotating elements (2.11.4-3.10.5-4.9.5) of said handpieces (2-3-4) have the outer surface provided with a plurality of spherical cap-shaped recesses, suitable to create a suction effect when pressed on the epidermis of the patient; the outer surface of said rotating elements (2.11.4-3.10.5-4.9.5) being provided with a surface treatment suitable to obtain a predetermined surface roughness in order to obtain the desired exfoliating effect on the epidermis of the patient.

## Patentansprüche

1. Integrierte Vorrichtung zur nicht-invasiven, personalisierten Behandlung von Hautmakeln, die von einem Bediener betätigt werden kann, um eine Behandlung am Körper und/oder im Gesicht eines Patienten zur Verringerung von Muskelkontrakturen, Cellulite, Dehnungsstreifen, Falten und anderen Makeln durchzuführen, wobei die Vorrichtung eine tragbare Konsole (1) aufweist, die im oberen Teil mit mehreren Aussparungen (1.1-1.2-1.3) versehen ist, die jeweils geeignet sind, ein entsprechenden Handstück (2-3-4) aufzunehmen, das den Bediener ergriffen werden kann, um einen Schritt einer vorgegebenen Behandlung am Körper und/oder im Gesicht des Patienten durchzuführen; wobei die Handstücke (2-3-4) aufweisen:
- ein erstes Handstück (2), das geeignet ist, eine Perkussionsbehandlung am Körper und/oder am Gesicht eines Patienten mittels eines Rotor (2.11) durchzuführen, der eine Mehrzahl rotierender Elemente (2.11.4) aufweist, die auf einer entsprechenden Mehrzahl von äquidistanten Halbachsen (2.11.2) am Umfang einer Ringmutter (2.11.1) installiert sind; wobei der Rotor (2.11) zwischen einem beweglichen Kopf (2.2), der mit einem Punktlaser und mit einer Mikrokamera ausgestattet ist, des ersten Handstücks (2) und einem Winkelträger (2.5) installiert ist; wobei der Kopf (2.2) mit einer elastischen Membran versehen ist, die geeignet ist, es zu ermöglichen, die Perkussion durchzuführen, wobei der direkte Kontakt zwischen den rotierenden Elementen (2.11.4) und der Epidermis des Patienten vermieden wird; wobei der Winkelträger (2.5) mittels eines geeigneten Einsatzes (2.6) an einem Elektromotor (2.7) montiert ist, der die Perkussionsfrequenz des Rotors (2.11) steuert; wobei der Elektromotor (2.7) durch ein spezielles Kabel, das mit dem ersten Handstück (2) mittels üblicher Verbindungsmittel (2.8), die mit der Konsole (1) verbunden sind, verbunden ist, mit Leistung versorgt wird; wobei der Winkelträger (2.5), der Einsatz (2.6), der Elektromotor (2.7) und die Verbindungsmittel (2.8) in einem Paar von Halbgriffen (2.9-2.10) angeordnet sind, die geeignet sind, gegenseitig in Eingriff gebracht zu werden, um den Griff zum Ergreifen des ersten Handstücks (2) zu bilden; wobei das erste Handstück (2) geeignet ist, ein Mikrotrauma an der Faltenfurche zu verursachen und dadurch die natürliche Erholung und damit eine Verringerung der Faltentiefe zu erleichtern;
- ein zweites Handstück (3), das geeignet ist, eine ablative Mikrovibrationsbehandlung am Körper des Patienten mittels eines Rotor (3.10) durchzuführen, der eine Mehrzahl rotierender Elemente (3.10.5) aufweist, die geeignet sind, in einer konvergierenden oder divergierenden Richtung in Bezug aufeinander zu vibrieren und zu rotieren, je nach der Anordnung einer Mehrzahl von Pins (3.10.9), an denen die rotierenden Elemente (3.10.5) montiert sind; wobei sich die Pins (3.10.9) von einer oberen Ringmutter (3.10.2) zu einer mittleren Ringmutter (3.10.4) und von der mittleren Ringmutter (3.10.4) zu einer unteren Ringmutter (3.10.6) erstrecken; wobei die Drehung der mittleren Ringmutter (3.10.4) durch den Bediener geeignet ist, die Konfiguration der Pins (3.10.9) zu ändern, um sicherzustellen, dass die rotierenden Elemente (3.10.5) zu der mittleren Ringmutter (3.10.4) hin konvergieren oder von ihr weg divergieren; wobei der Rotor (3.10) geeignet ist, mit einer vorgegebenen Frequenz um eine obere Halbachse (3.10.3) und eine untere Halbachse (3.10.8) zu rotieren; wobei der Rotor (3.10) in eine Schale (3.4) eingesetzt ist, die mit einer offenen und mit einer geschlossenen Seite versehen ist; wobei die offene Seite den Kontakt zwischen den rotierenden Elementen (3.10.5) und dem Körper des Patienten ermöglicht; wobei die geschlossene Seite dem Bediener zugewandt ist und ein Paar von Anzeigern (3.3) aufweist, die geeignet sind, dem Bediener die konvergierende oder divergierende Richtung der rotierenden Elemente (3.10.5) auf dem Körper des Patienten anzuzeigen; wobei der Rotor (3.10) am oberen Teil mittels eines Pins (3.10.1) mit einem Lager (3.2) und schließlich mit einer oberen Kappe (3.1) des zweiten Handstücks (3) verbunden ist; wobei der Rotor (3.10) am unteren Teil mit einem Elektromotor (3.5) verbunden ist, der in einen Griff (3.6) integriert ist; wobei der Elektromotor (3.5) geeignet ist, die Rotations- und Vibrationsfrequenz des Rotors (3.10) zu bestimmen und durch ein spezielles Kabel mit Leistung versorgt wird, das mit dem zweiten Handstück (3) mittels üblicher Verbindungsmittel (3.7) verbunden ist, die in einer unteren Kappe (3.8) enthalten sind, die mit dem Griff (3.6) verbunden ist, wobei das Leistungsversorgungskabel mit der Konsole (1) verbunden ist; wobei das zweite Handstück (3) geeignet ist, das Hautgewebe zu dehnen oder zu verdichten, um Makel wie etwa Falten, Dehnungsstreifen und Narben zu mildern und auch einen Peelingeffekt, der "Stretching" genannt wird, zu erzielen;
- ein drittes Handstück (4), das geeignet ist, eine komprimierende Mikrovibrationsbehandlung auf dem Gesicht eines Patienten mittels eines Rotors (4.9) durchzuführen, der eine Mehrzahl rotierender Elemente (4.9.5) aufweist, die geeignet sind, zu vibrieren und um eine Mehrzahl von Pins (4.9.4) zu rotieren, an denen die rotierenden Elemente (4.9.5) montiert sind; wobei sich die Pins (4.9.4) von einer oberen Ringmutter (4.9.2) zu einer unteren Ringmutter (4.9.6) erstrecken; wobei der Rotor (4.9) geeignet ist, mit einer vorgegebenen Frequenz um eine Rotationsachse (4.9.3) zu rotieren; wobei der Rotor (4.9) in eine Schale (4.4) eingesetzt ist, die mit einer offenen und einer geschlossenen Seite versehen ist; wobei die offene Seite den Kontakt zwischen den rotierenden Elementen (4.9.5) und dem Körper des Patienten ermöglicht; wobei die geschlossene Seite dem Bediener zugewandt ist und einen Leichtdruckanzeiger (4.4.1), die geeignet ist, dem/der Bediener(in) den Druck zu signalisieren, mit dem er/sie das Handstück (4) auf das Gesicht des Patienten drückt; wobei der Rotor (4.9) am oberen Teil mittels eines Pins (4.9.1) mit einem Lager (4.2) und schließlich mit einer oberen Kappe (4.1) des dritten Handstücks (4) verbunden ist; wobei der Rotor (4.9) am unteren Teil mittels einer Verbindung (4.9.7) mit einem in einen Griff (4.5) integrierten Elektromotor (4.6) verbunden ist; wobei der Elektromotor (3.5) dazu geeignet ist, die Rotations- und Vibrationsfrequenz des Rotors (4.9) zu bestimmen und durch ein spezielles Kabel mit Leistung versorgt wird, das mit dem dritten Handstück (4) mittels üblicher Verbindungsmittel (4.8) verbunden ist, die in einer unteren Kappe (4.7) enthalten sind, die mit dem Griff (4.5) verbunden ist, wobei das Leistungsversorgungskabel mit der Konsole (1) verbunden ist; wobei das dritte Handstück (4) dazu geeignet ist, die Poren der Epidermis des Patienten zu erweitern;
- zumindest eine Vorrichtung zur Einstellung der Rotation und der Vibration, die in jedes Handstück (2-3-4) integriert und geeignet ist, die Frequenz der Rotationen und Vibrationen der Rotoren (2.11-3.10-4.9) der Handstücke (2-3-4) zu steuern;
- zumindest einen Sensor für die Absorption elektrischer Energie für jedes Handstück (2-3-4), der dazu geeignet ist, die Menge an elektrischer Energie zu bestimmen, die durch jedes Handstück (2-3-4) absorbiert wird, um die gewünschte Rotation und Vibration der entsprechenden Rotoren (2.11-3.10-4.9) zu erhalten; wobei der Sensor für die Absorption elektrischer Energie dazu geeignet ist, die detektierten Daten zu übermitteln an
- eine Steuerungseinheit, die mit einer spezifischen Software programmiert ist, die geeignet ist, die von den Sensoren für die Absorption elektrischer Energie stammenden Daten zu verarbeiten und folglich in Echtzeit auf die Vorrichtungen zur Einstellung der Frequenz eines jeden Handstücks (2-3-4) einzuwirken, um eine unmittelbare Änderung der Rotations- und Vibrationsfrequenz der Rotoren (2.11-3.10-4.9) innerhalb eines vorgegebenen Bereichs zu erhalten, der zwischen 20 Hz und 700 Hz, vorzugsweise zwischen 40 Hz und 355 Hz, liegt, und zwar in Abhängigkeit von der mechanischen Festigkeit, die das Hautgewebe des Patienten in Kontakt mit einem der Handstücke (2-3-4) aufweist;
- eine Kommunikationsschnittstelle (5), die vorzugsweise in die Konsole (1) integriert ist und geeignet ist, es einem Bediener zu ermöglichen, in Echtzeit Informationen über die laufende Behandlung zu empfangen und die entsprechenden Parameter einzustellen, einschließlich der Frequenz der Vibrationen und der Rotationen, die dem Patienten über die Handstücke (2-3-4) verabreicht werden sollen, wobei die Vorrichtung außerdem Mittel zum Halten der Handstücke (2-3-4-7) enthält, die dazu geeignet sind, die Beschränkung zwischen einer Aussparung (1.1-1.2-1.3) und dem entsprechenden Handstück (2-3-4-7) nur in der Reihenfolge freizugeben, die durch die durchgeführte Therapie vorgegeben ist.

2. Integrierte Vorrichtung zur nicht-invasiven, personalisierten Behandlung von Hautmakeln nach dem vorhergehenden Anspruch 1, **dadurch gekennzeichnet, dass** sie zumindest ein viertes Handstück (7) aufweist, das dazu geeignet ist, eine Behandlung zum vibrierenden Transport von Wirkstoffen tief in den Körper und/oder auf das Gesicht eines Patienten mittels eines Kopfes (7.1) durchzuführen, der dazu geeignet ist, unter Ausnutzung von elektrischen Rechteckpulsen mit einer vorgegebenen Frequenz zu vibrieren, angetrieben durch einen Elektromotor (7.4), der in einen Griff (7.3) eingesetzt ist, der im oberen Teil mit einem Träger (7.2) verbunden ist, von dem der vibrierende Kopf (7.1) oben vorspringt; wobei der Elektromotor (7.4) durch ein spezielles Kabel mit Leistung versorgt wird, das mit dem vierten Handstück (7) mittels üblicher Verbindungsmittel (7.6) verbunden ist, die in einer unteren Kappe (7.5) enthalten sind, die mit dem Griff (7.3) verbunden ist, wobei das Leistungsversorgungskabel mit der Konsole (1) verbunden ist.

3. Integrierte Vorrichtung zur nicht-invasiven, personalisierten Behandlung von Hautmakeln nach dem vorhergehenden Anspruch 2, **dadurch gekennzeichnet, dass** das vierte Handstück (7) ein Wirkstoffreservoir aufweist, das dazu geeignet ist, die gewünschte Menge an Creme, Gel oder Mousse durch Drücken eines entsprechenden Knopfes durch den Bediener auf den Körper des Patienten abzugeben.

4. Integrierte Vorrichtung zur nicht-invasiven, personalisierten Behandlung von Hautmakeln nach einem der vorhergehenden Ansprüche, **dadurch gekennzeichnet, dass** die Handstücke (2- 3-4-7) mit einem internen Widerstand versehen sind, der dazu geeignet ist, den Teil des Handstücks (2-3-4-7), der mit dem Körper oder dem Gesicht des Patienten in Kontakt steht, auf eine vorgegebene Temperatur zu erwärmen, wenn das Handstück (2-3-4-7) in Gebrauch ist.

5. Integrierte Vorrichtung zur nicht-invasiven, personalisierten Behandlung von Hautmakeln nach einem der vorhergehenden Ansprüche 1 bis 3, **dadurch gekennzeichnet, dass** jede Aussparung (1.1-1.2-1.3) mit einem internen Widerstand versehen ist, der dazu geeignet ist, die entsprechenden Handstücke (2-3-4-7) zu erwärmen, bevor sie mit dem Körper oder dem Gesicht des Patienten in Kontakt gebracht werden.

6. Integrierte Vorrichtung zur nicht-invasiven, personalisierten Behandlung von Hautmakeln nach einem der vorhergehenden Ansprüche, **dadurch gekennzeichnet, dass** jedes Handstück (2- 3-4-7) mit einem Temperatursensor versehen ist, der dazu geeignet ist, der Steuerungseinheit der Vorrichtung die Temperaturveränderung der Hautgewebe des Patienten ab Beginn der Behandlung zu übermitteln, wobei die Steuerungseinheit den Wert dieser Veränderung mittels der Kommunikationsschnittstelle (5) übermittelt.

7. Integrierte Vorrichtung zur nicht-invasiven, personalisierten Behandlung von Hautmakeln nach einem der vorhergehenden Ansprüche, **dadurch gekennzeichnet, dass** die rotierenden Elemente (2.11.4-3.10.5-4.9.5) der Handstücke (2-3-4) aus Kugeln, Ovoiden oder Zylindern bestehen, die so angeordnet sind, dass sie eine waben- oder V-förmige Form bilden; wobei die rotierenden Elemente (2.11.4-3.10.5-4.9.5) aus nichtallergenem Silikon oder Gel mit einer Härte zwischen 5 Shore und 100 Shore, vorzugsweise 35 Shore, hergestellt sind.

8. Integrierte Vorrichtung zur nicht-invasiven, personalisierten Behandlung von Hautmakeln nach einem der vorhergehenden Ansprüche, **dadurch gekennzeichnet, dass** die äußere Oberfläche der rotierenden Elemente (2.11.4-3.10.5-4.9.5) der Handstücke (2-3-4) mit einer Mehrzahl kugelförmiger, kappenförmiger Aussparungen versehen ist, die dazu geeignet sind, einen Saugeffekt zu erzeugen, wenn sie auf die Epidermis des Patienten gedrückt werden; wobei die äußere Oberfläche der rotierenden Elemente (2.11.4- 3.10.5-4.9.5) mit einer Oberflächenbehandlung versehen ist, die dazu geeignet ist, eine vorgegebene Oberflächenrauhigkeit zu erhalten, um den gewünschten Peelingeffekt auf der Epidermis des Patienten zu erzielen.

## Revendications

1. Dispositif intégré pour le traitement personnalisé non invasif d'imperfections cutanées, apte à être utilisé par un opérateur pour réaliser un traitement sur le corps et/ou sur le visage d'un patient pour la réduction des contractures musculaires, de la cellulite, des vergetures, des rides et autres imperfections, ledit dispositif comprenant une console portable (1), dotée, au niveau de la partie supérieure, d'une pluralité de cavités (1.1-1.2-1.3) chacune apte à loger une pièce à main correspondante (2-3-4) apte à être saisie par ledit opérateur pour réaliser une étape d'un traitement préétabli sur le corps et/ou le visage dudit patient ; lesdites pièces à main (2-3-4) comprenant :
- une première pièce à main (2) apte à effectuer un traitement par percussion sur le corps et/ou le visage d'un patient au moyen d'un rotor (2.11) comprenant une pluralité d'éléments rotatifs (2.11.4) installés sur une pluralité correspondante de semi-axes équidistants (2.11.2) sur la circonférence d'un écrou à anneau (2.11.1) ; ledit rotor (2.11) étant installé entre une tête mobile (2.2), dotée d'un laser ponctuel et d'une micro-caméra, de ladite première pièce à main (2) et un support angulaire (2.5) ; ladite tête (2.2) étant dotée d'une membrane élastique apte à permettre la réalisation de la percussion, en évitant le contact direct entre les éléments rotatifs (2.11.4) et l'épiderme du patient ; ledit support angulaire (2.5) étant monté, au moyen d'un insert approprié (2.6), sur un moteur électrique (2.7) qui commande la fréquence de percussion dudit rotor (2.11) ; ledit moteur électrique (2.7) étant alimenté par un câble spécial, raccordé à ladite première pièce à main (2) au moyen d'un moyen de raccordement (2.8) commun, raccordé à ladite console (1) ; ledit support angulaire (2.5), ledit insert (2.6), ledit moteur électrique (2.7) et ledit moyen de raccordement (2.8) étant agencés en une paire de semi-poignées (2.9-2.10) aptes à se mettre en prise mutuellement pour former la poignée pour saisir ladite première pièce à main (2) ; ladite première pièce à main (2) étant apte à provoquer un microtraumatisme au niveau du sillon de rides, facilitant ainsi la récupération naturelle et par conséquent une réduction de la profondeur des rides ;
- une deuxième pièce à main (3) apte à effectuer un traitement par micro-vibration ablative sur le corps du patient au moyen d'un rotor (3.10) comprenant une pluralité d'éléments rotatifs (3.10.5) aptes à vibrer et pivoter dans une direction mutuellement convergente ou divergente en fonction de l'agencement d'une pluralité de goupilles (3.10.9) sur lesquelles lesdits éléments rotatifs (3.10.5) sont montés ; lesdites goupilles (3.10.9) s'étendant à partir d'un écrou à anneau supérieur (3.10.2) jusqu'à un écrou à anneau intermédiaire (3.10.4) et à partir dudit écrou à anneau intermédiaire (3.10.4) jusqu'à un écrou à anneau inférieur (3.10.6) ; la rotation dudit écrou à anneau intermédiaire (3.10.4) par ledit opérateur étant apte à modifier la configuration desdites goupilles (3.10.9) de manière à assurer que lesdits éléments rotatifs (3.10.5) convergent ou divergent en direction ou à partir dudit écrou à anneau intermédiaire (3.10.4) ; ledit rotor (3.10) étant apte à pivoter, à une fréquence préétablie, autour d'un semi-axe supérieur (3.10.3) et d'un semi-axe inférieur (3.10.8) ; ledit rotor (3.10) étant inséré dans un carter (3.4) doté d'un côté ouvert et d'un côté fermé ; ledit côté ouvert permettant le contact entre lesdits éléments rotatifs (3.10.5) et le corps dudit patient ; ledit côté fermé étant dirigé vers l'opérateur et comprenant une paire d'indicateurs lumineux (3.3) aptes à signaler à l'opérateur la direction convergente ou divergente des éléments rotatifs (3.10.5) sur le corps du patient ; ledit rotor (3.10) étant raccordé, au niveau de la partie supérieure au moyen d'une goupille (3.10.1), à un roulement (3.2) et finalement à un capuchon supérieur (3.1) de ladite deuxième pièce à main (3) ; ledit rotor (3.10) étant raccordé, au niveau de la partie inférieure, à un moteur électrique (3.5) intégré dans une poignée (3.6) ; ledit moteur électrique (3.5) étant apte à déterminer la fréquence de rotation et de vibration dudit rotor (3.10) et étant alimenté par un câble spécial, raccordé à ladite deuxième pièce à main (3) au moyen d'un moyen de raccordement (3.7) commun inclus à l'intérieur d'un capuchon inférieur (3.8) raccordé à ladite poignée (3.6), ledit câble d'alimentation étant raccordé à ladite console (1) ; ladite deuxième pièce à main (3) étant apte à dilater ou compacter le tissu cutané, atténuant les imperfections telles que les rides, les vergetures et les cicatrices et obtenant également un effet exfoliant appelé « étirement » ;
- une troisième pièce à main (4) apte à effectuer un traitement par microvibration compressive sur le visage d'un patient au moyen d'un rotor (4.9) comprenant une pluralité d'éléments rotatifs (4.9.5) aptes à vibrer et pivoter autour d'une pluralité de goupilles (4.9.4) sur lesquelles lesdits éléments rotatifs (4.9.5) sont montés ; lesdites goupilles (4.9.4) s'étendant à partir d'un écrou à anneau supérieur (4.9.2) jusqu'à un écrou à anneau inférieur (4.9.6) ; ledit rotor (4.9) étant apte à pivoter, à une fréquence préétablie, autour d'un axe de rotation (4.9.3) ; ledit rotor (4.9) étant inséré dans un carter (4.4) doté d'un côté ouvert et d'un côté fermé ; ledit côté ouvert permettant le contact entre lesdits éléments rotatifs (4.9.5) et le corps dudit patient ; ledit côté fermé étant dirigé vers l'opérateur et comprenant un indicateur de pression lumineux (4.4.1) apte à signaler à l'opérateur la pression avec laquelle il/elle presse la pièce à main (4) sur le visage du patient ; ledit rotor (4.9) étant raccordé, au niveau de la partie supérieure au moyen d'une goupille (4.9.1), à un roulement (4.2) et finalement à un capuchon supérieur (4.1) de ladite troisième pièce à main (4) ; ledit rotor (4.9) étant raccordé, au niveau de la partie inférieure au moyen d'un joint (4.9.7), à un moteur électrique (4.6) intégré dans une poignée (4.5) ; ledit moteur électrique (3.5) étant apte à déterminer la fréquence de rotation et de vibration dudit rotor (4.9) et étant alimenté par un câble spécial, raccordé à ladite troisième pièce à main (4) au moyen d'un moyen de raccordement (4.8) commun inclus à l'intérieur d'un capuchon inférieur (4.7) raccordé à ladite poignée (4.5), ledit câble d'alimentation étant raccordé à ladite console (1) ; ladite troisième pièce à main (4) étant apte à dilater les pores de l'épiderme du patient ;
- au moins un dispositif d'ajustement des rotations et des vibrations, intégré dans chaque pièce à main (2-3-4) apte à commander la fréquence desdites rotations et vibrations desdits rotors (2.11-3.10-4.9) desdites pièces à main (2-3-4) ;
- au moins un capteur d'absorption d'énergie électrique pour chaque pièce à main (2-3-4), apte à détecter la quantité d'énergie absorbée par chaque pièce à main (2-3-4) pour obtenir la rotation et la vibration souhaitées des rotors relatifs (2.11-3.10-4.9) ; ledit capteur d'absorption d'énergie électrique étant apte à communiquer les données détectées à
- une unité de commande programmée avec un logiciel dédié apte au traitement des données provenant desdits capteurs d'absorption d'énergie électrique et à agir en conséquence, en temps réel, sur lesdits dispositifs d'ajustement de fréquence de chaque pièce à main (2-3-4) pour obtenir une variation immédiate de la fréquence de rotation et de vibration desdits rotors (2.11-3.10-4.9), à l'intérieur d'une plage préétablie comprise entre 20 Hz et 700 Hz, de préférence entre 40 Hz et 355 Hz, en fonction de la résistance mécanique fournie par le tissu cutané du patient au contact de l'une desdites pièces à main (2-3-4) ;
- une interface de communication (5), de préférence intégrée dans ladite console (1), apte à permettre à un opérateur de recevoir en temps réel des informations relatives au traitement en cours et à régler les paramètres respectifs, y compris la fréquence des vibrations et des rotations à administrer au patient par l'intermédiaire desdites pièces à main (2-3-4), dans lequel le dispositif comprend en outre un moyen de retenue desdites pièces à main (2-3-4-7) apte à libérer la contrainte entre une cavité (1.1-1.2-1.3) et la pièce à main correspondante (2-3-4-7) uniquement dans l'ordre préétabli par la thérapie qui est réalisée.

2. Dispositif intégré pour le traitement personnalisé non invasif d'imperfections cutanées, selon la revendication 1, **caractérisé en ce qu'**il comprend au moins une quatrième pièce à main (7) apte à réaliser un traitement pour le vibro-transport des ingrédients actifs profond dans le corps et/ ou sur le visage d'un patient au moyen d'une tête (7.1) apte à vibrer en utilisant des impulsions électriques à ondes carrées, à une fréquence préétablie entraînée par un moteur électrique (7.4) inséré dans une poignée (7.3) raccordée, au niveau de la partie supérieure, à un support (7.2) à partir duquel ladite tête vibrante (7.1) fait saillie au-dessus ; ledit moteur électrique (7.4) étant alimenté par un câble spécial, raccordé à ladite quatrième pièce à main (7) au moyen d'un moyen de raccordement (7.6) commun inclus à l'intérieur d'un capuchon inférieur (7.5) raccordé à ladite poignée (7.3), ledit câble d'alimentation étant raccordé à ladite console (1).

3. Dispositif intégré pour le traitement personnalisé non invasif d'imperfections cutanées, selon la revendication 2 précédente, **caractérisé en ce que** ladite quatrième pièce à main (7) comprend un réservoir de principe actif apte à libérer la quantité souhaitée de crème, gel, ou mousse sur le corps du patient sous l'effet d'une pression sur un bouton approprié par ledit opérateur.

4. Dispositif intégré pour le traitement personnalisé non invasif d'imperfections cutanées, selon l'une des revendications précédentes, **caractérisé en ce que** lesdites pièces à main (2-3-4-7) sont dotées d'une résistance interne apte à chauffer la partie de ladite pièce à main (2-3-4-7) qui est en contact avec le corps ou avec le visage du patient lorsque ladite pièce à main (2-3-4-7) est utilisée, jusqu'à une température préétablie.

5. Dispositif intégré pour le traitement personnalisé non invasif d'imperfections cutanées, selon l'une des revendications précédentes 1 à 3, **caractérisé en ce que** chaque cavité (1.1-1.2-1.3) est dotée d'une résistance interne apte à chauffer les pièces à main correspondantes (2-3-4-7) avant qu'elles ne soient placées au contact du corps ou du visage du patient.

6. Dispositif intégré pour le traitement personnalisé non invasif d'imperfections cutanées, selon l'une des revendications précédentes, **caractérisé en ce que** chaque pièce à main (2-3-4-7) est dotée d'un capteur de température apte à communiquer, à l'unité de commande dudit dispositif, la variation de température des tissus cutanés du patient depuis le début du traitement, ladite unité de commande communiquant la valeur de ladite variation au moyen de ladite interface de communication (5) .

7. Dispositif intégré pour le traitement personnalisé non invasif d'imperfections cutanées, selon l'une des revendications précédentes **caractérisé en ce que** lesdits éléments rotatifs (2.11.4-3.10.5-4.9.5) desdites pièces à main (2-3-4) sont constitués de balles, d'ovoïdes ou de cylindres, agencés pour former une forme de type nid-d'abeilles ou de type V ; lesdits éléments rotatifs (2.11.4-3.10.5-4.9.5) étant composés de silicone non allergénique ou d'un gel ayant une dureté comprise entre 5 shores et 100 shores, de préférence de 35 shores.

8. Dispositif intégré pour le traitement personnalisé non invasif d'imperfections cutanées, selon l'une des revendications précédentes, **caractérisé en ce que** lesdits éléments rotatifs (2.11.4-3.10.5-4.9.5) desdites pièces à main (2-3-4) ont la surface externe dotée d'une pluralité de cavités en forme de capuchon sphérique, aptes à créer un effet de succion lors d'une pression sur l'épiderme du patient ; la surface externe desdits éléments rotatifs (2.11.4-3.10.5-4.9.5) étant dotée d'un traitement de surface apte à obtenir une rugosité de surface prédéterminée de manière à obtenir l'effet exfoliant souhaité sur l'épiderme du patient.
